# EUROPÄISCHE PATENTSCHRIFT

(11) **EP 3 454 767 B1**
(45) Veröffentlichungstag und Bekanntmachung des Hinweises auf die Patenterteilung: **17.01.2024**
(21) Anmeldenummer: 17721716.3
(22) Anmeldetag: 08.05.2017
(51) Int. Cl.: A61B 17/70

(54) **MEDIZINTECHNISCHES INSTRUMENT ZUR PROVISORISCHEN FIXIERUNG EINER POLYAXIALEN PEDIKELSCHRAUBE**
MEDICAL INSTRUMENT FOR PROVISIONALLY FASTENING A POLYAXIAL PEDICLE SCREW
INSTRUMENT À USAGE MÉDICAL DE FIXATION PROVISOIRE D'UNE VIS DE PÉDICULAIRE POLYAXIALE

(30) Priorität: 09.05.2016 DE 102016108504
(43) Veröffentlichungstag der Anmeldung: 20.03.2019
(73) Patentinhaber: Aesculap AG, 78532 Tuttlingen (DE)
(72) Erfinder: LINDNER, Stephan, 78573 Wurmlingen (DE)
(74) Vertreter: Winter, Brandl - Partnerschaft mbB
(86) Internationale Anmeldenummer: PCT/EP2017/060919
(87) Internationale Veröffentlichungsnummer: WO 2017/194470

(56) Entgegenhaltungen:
- EP-A1- 2 832 308
- US-A1- 2009 228 054
- US-A1- 2014 277 137
- US-A1- 2016 113 682

## Beschreibung

Die vorliegende Erfindung betrifft ein medizintechnisches Instrument zur provisorischen Fixierung einer polyaxialen Pedikelschraube mit einem Gewindeschaft mit einem proximal daran ausgebildeten Schaftkopf, an dem eine Aufnahmehülse drehbar und/oder schwenkbar angeordnet ist, wobei in der Aufnahmehülse ein Klemmstempel auf den Schaftkopf einwirkend gelagert ist, derart, dass durch relatives Verspannen von Aufnahmehülse und Klemmstempel die Aufnahmehülse am Schaftkopf lagefixierbar ist, wobei das Instrument eine Koppeleinheit zum lösbaren Ineingriffbringen mit der Aufnahmehülse und einen Druckstößel, auch als Insertpusher bezeichnet, zum Ausüben einer Anpresskraft auf den Klemmstempel in distale Richtung aufweist, wobei die Koppeleinheit und der Druckstößel in einer Axialrichtung relativ zueinander bewegbar sind.

Pedikelschrauben dienen prinzipiell zur dorsalen Stabilisierung der Wirbelsäule bei Frakturen, Tumoren, Entzündungen, Deformitäten und degenerativ bedingten Instabilitäten mittels transpedikulärer Verschraubung. Dabei werden Pedikelschrauben in den Pedikeln jeweils benachbarter Wirbel platziert, worauf eine winkelstabile Verbindung zwischen den jeweils axial übereinander sich anordnenden Pedikelschrauben und einem axial sich erstreckenden Längsträger oder Steg geschaffen wird. Die Pedikelschrauben und Längsträger bilden dabei ein Wirbel-Stabilisierungssystem.

Hierfür hat eine Pedikelschraube in der Regel einen axialen, schaftartigen Außengewindeabschnitt, an den sich schraubenkopfseitig eine sogenannte Tulpe anschließt. Diese bildet konstruktiv eine U-förmig längs geschlitzte/getunnelte Aufnahmehülse mit Innengewinde, wobei die beiden sich radial gegenüberliegenden Längsschlitze jeweils einen Schlitzspalt vorbestimmter Spaltbreite definieren. In die parallel zueinander verlaufenden Längsschlitze ist der Längsträger/Stab quer eingelegt, der mittels eines Verriegelungselements, zum Beispiel in Form einer Madenschraube, Gewindemutter oder Setscrew, die in das Innengewinde eingedreht ist, fixiert wird.

Grundsätzlich werden zwei Grundarten von Pedikelschrauben unterschieden, nämlich monoaxiale sowie polyaxiale Pedikelschrauben. Im Fall einer monoaxialen Pedikelschraube sind der Außengewindeabschnitt/Schaft und die Tulpe/Aufnahmehülse einstückig miteinander ausgebildet. Eine polyaxiale Pedikelschraube hat hingegen einen als separates Bauteil gefertigten Außengewindeschaft mit einem zumeist kugelförmigen oder (semi-) sphärischen Schraubenkopf, der von der Aufnahmehülse/Tulpe relativ verschwenkbar umgriffen und gleichzeitig im Übergangsbereich zwischen Kopf und Schaft hintergriffen wird. Auf diese Weise kann die Aufnahmehülse/Tulpe nach Versenken des Außengewindeschafts im Pedikelkanal eines Wirbels relativ dazu verschwenkt und/oder verdreht werden, um eine gewünschte Lage und Ausrichtung im Wesentlichen unabhängig zur Ausrichtung des Schafts zu erhalten. Die Hinterschneidung verhindert dabei, dass die Aufnahmehülse/Tulpe vom Schaftkopf abgezogen werden kann. Anschließend wird die Aufnahmehülse/Tulpe mittels des Verriegelungselements/Setscrew bei zwischengelagertem Steg (Ein-Schrauben-Prinzip) oder durch eine zusätzliche Schraube/Schraubenmutter (Mehr-Schrauben-Prinzip) am Schraubenkopf lagefixiert.

Pedikelschrauben werden von einem Operateur in den Pedikelkanal eines Wirbels eingesetzt bzw. durch Verschrauben verankert. Dabei richtet der Operateur die Schrauben aufgrund der Orientierung des Pedikelkanals aus. Wenn die Schrauben gesetzt sind, wird der vorstehend genannte Längsträger oder Steg der richtigen Länge ausgewählt und ggf. in seiner Längskrümmung an die Pedikelschrauben sowie deren jeweilige Position angepasst. Der Längsträger soll dabei in den Tulpen der Pedikelschrauben platziert werden.

Die zum Gewindeschaft bewegliche Lagerung der Aufnahmehülse einer polyaxialen Pedikelschraube erleichtert es dem Operateur, den Längsträger lateral in die Aufnahmehülse einzusetzen/einzuführen. Ist der Operateur mit dem Sitz des Längsträgers und der Aufnahmehülse zufrieden, verriegelt er die Pedikelschraube mittels des Verriegelungselements, vorzugsweise der Madenschraube (prinzipiell auch Setzschraube beliebiger Ausgestaltung genannt). Die Polyaxialverriegelung (Lagefixation der Aufnahmehülse bezüglich des Schafts) sowie die Längsträgerverklemmung werden im Fall des vorstehend genannten Ein-Schrauben-Prinzips beim Anziehen der einen Setzschraube in einem einzigen Montageschritt verriegelt.

Bei Polyaxialschrauben ist es aufgrund der Beweglichkeit (Gelenk) zwischen dem Schraubenschaft und der Aufnahmehülse während des Implantierungsvorgangs in der Regel nicht möglich, Kräfte von der Aufnahmehülse aus in den Wirbel einzuleiten, um diesen beispielsweise manipulieren zu können. Dies ist insbesondere bei Repositionsmanövern im Fall von Frakturen oder Spondylolisthesen und zum Teil bei Kompressionen oder Distraktionen jedoch erforderlich. Hierfür werden daher die monoaxialen Pedikelschrauben verwendet, bei welchen die Aufnahmehülse zum Schraubenschaft starr ist. Diese monoaxialen Pedikelschrauben haben jedoch wiederum den Nachteil, dass der Längsträger nur schwer in die Tulpe/Aufnahmehülse mehrerer monoaxialen Pedikelschrauben lateral eingeführt werden kann.

Bei vielen Pedikelschrauben gemäß vorstehend beschriebenem Aufbau sind die Fixiermittel/Verriegelungselemente (Schrauben) im Wesentlichen selbsthemmend, um nach Implantierung kein ungewolltes Lösen der Längsträger von den Pedikelschrauben zu riskieren. Darüber hinaus sind die Fixationskräfte zwischen Pedikelschraube und Längsträger beträchtlich, da das gesamte System großen Belastungen standhalten muss, ohne dass sich die eingestellte Lagebeziehung zwischen Gewindeschaft, Aufnahmehülse und Längsträger verändern darf. Daraus ergibt sich, dass die Klemmkräfte zwischen dem Schaftkopf und der Aufnahmehülse ebenfalls sehr hoch sind, sodass der sich dazwischen ausbildende Reibschluss häufig selbst dann nicht frei kommt, wenn das Fixiermittel gelöst ist. Diese Notwendigkeiten verursachen indessen auch Probleme während des Implantierungsvorgangs.

Solange die Aufnahmehülse nicht relativ zum Schaftkopf fixiert ist, zum Beispiel durch Reibschluss fest mit dem Schaftkopf verbunden ist, kann über diese keine Justierkraft auf den Wirbel übertragen werden. Im Fall einer polyaxialen Pedikelschraube nach dem Ein-Schrauben-Prinzip müsste demnach zunächst ein Längsträger eingelegt und dann das Verriegelungselement, vorzugsweise die Madenschraube, festgezogen werden, um schließlich eine Justierkraft auf den Wirbel aufbringen zu können. Dies wäre jedoch technisch nicht sinnvoll.

Hat ein Operateur erst einmal das Verriegelungselement/Setscrew mit Kraft festgezogen und ist ggf. nachträglich eine Nachjustierung der Wirbelposition erforderlich, ist die hierdurch festgelegte Lagebeziehung nicht, oder nur mit großem Aufwand wieder veränderbar. In anderen Worten ausgedrückt, müsste hierfür der Operateur die mit großer Kraft festgezogene(n) Madenschraube(n) entgegen deren Selbsthemmwirkung wieder lösen, ohne hierbei den im Wirbel bereits verankerten Außengewindeabschnitt mit zu lösen oder gar herauszubrechen. Selbst wenn die Madenschraube problemlos gelöst werden kann, hat sich zwischen dem Schaftkopf und der Aufnahmehülse ggf. ein selbsthemmender Reibschluss ausgebildet. Dieser könnte auch bei gelöster Madenschraube nur durch erheblichen Kraftaufwand (Schläge auf die Aufnahmehülse, etc.) freikommen. Des Weiteren wird durch das nachträgliche Lösen des Verriegelungselements dessen Selbsthemmwirkung ggf. beeinträchtigt, sodass die Funktionsfähigkeit der Pedikelschraube nicht mehr gewährleistet ist.

Aus der WO 2013/034351 A1 ist eine Pedikelschraube bekannt, bei der die Polyaxialität provisorisch fixierbar ist, so dass die Polyaxialität und der Steg/Längsträger zumindest temporär unabhängig verriegelt werden können, also nur die Polyaxialität fixiert wird, ohne dass der Steg fixiert wird. In dieser Veröffentlichung wird des Weiteren ein medizintechnisches Instrument oder Hilfsmittel zur provisorischen Fixierung der Polyaxialität einer Pedikelschraube offenbart. Die Pedikelschraube besteht im Wesentlichen aus einem geschraubten Schaft mit einem Schaftkopf, der von einer Aufnahmehülse umgeben ist. In dieser ist ein durch ein Verriegelungselement gegen den Schaftkopf vorspannbarer Stempel gelagert. Das Instrument weist ein erstes Bauteil in Form eines Druckstößels auf, um mit unmittelbar am Stempel vorgesehenen Angriffspunkten in drückenden Eingriff zu kommen. Es weist des Weiteren ein zweites Bauteil in Form eines hülsenförmigen Zugbauteils auf, das relativ zum ersten Bauteil bewegbar ist und ein Rastmittel hat, das mit der Aufnahmehülse in ziehenden Eingriff bringbar ist. Bei diesem Instrument ist von Nachteil, dass zum Abkoppeln des Zugbauteils dieses im Bereich der Aufnahmehülse der Pedikelschraube aufgespreizt werden muss. Dies ist aber unter Umständen kaum möglich, da ggf. knöcherne Strukturen hindernd im Weg stehen. Außerdem ist bei diesem Instrument ein versehentliches Entkoppeln von der Aufnahmehülse der Pedikelschraube nicht sicher auszuschließen.

Aus der US 2014/0277137 A1 ist ein medizintechnisches Instrument zur provisorischen Fixierung einer Polyaxialität einer polyaxialen Pedikelschraube bekannt, bei dem eine Außenkomponente ausgebildet ist, mit einer Aufnahmehülse der polyaxialen Pedikelschraube in Eingriff zu gelangen. Eine in der Außenkomponente aufgenommene Innenkomponente ist relativ zur Außenkomponente in Axialrichtung bewegbar, so dass die Innenkomponente auf ein Kompressionselement, das in einem proximalen Bereich der Aufnahmehülse aufgenommen ist, eine proximale Kraft ausüben kann und so die Aufnahmehülse lagefixieren kann. Bei diesem Instrument ist es von Nachteil, dass das Verriegelungselement/Setscrew im provisorisch fixierten Zustand (bei aktivierten Poly-Lock) nicht in das dafür vorgesehene Gewinde der Aufnahmehülse eingeschraubt werden kann. Des Weiteren kann der klein ausgebildete Hinterschnitt der Aufnahmehülse nur geringe Fixierungskräfte übertragen.

An dieser Stelle sei darauf hingewiesen, dass unter dem Begriff "provisorische Fixierung", insbesondere mit Blick auf die nachfolgend beschriebene Erfindung, nicht notwendiger Weise nur ein leichtes Anlegen/Verklemmen des Schaftkopfs gemeint sein soll, sondern insbesondere eine solche Verriegelungsmaßnahme zu verstehen ist, die temporär angewendet wird, sowie deren erzielbare Verriegelungseigenschaften wie die Einklemmkraft auf den Schaftkopf, etc. durchaus der dauerhaften Verriegelung entsprechen, dieser zumindest nahe kommen oder diese sogar übertreffen können. In anderen Worten ausgedrückt sollte die "provisorische" Verriegelung so dimensioniert sein, dass sie auch ein Verklemmen der Aufnahmehülse am Schaftkopf entsprechend dem dauerhaften Verriegelungselement bewirken kann. In diesem Fall wäre ggf. ein nachträgliches Lösen des Reibschlusses in Übereinstimmung mit dem Stand der Technik mit entsprechender Krafteinwirkung möglich, aber es besteht die Möglichkeit der Wirbelnachjustierung vor Fixierung des Längsträgers. Es ist also wünschenswert, die "provisorische" Verriegelung so zu dimensionieren, dass ausreichende Justierkräfte auf den Wirbel mittels der polyaxialen Pedikelschraube übertragbar sind, wobei der hierbei erzielte Reibschluss zwischen Schaftkopf und Aufnahmehülse wieder lösbar ist.

Ausgehend vom vorgenannten Stand der Technik liegt der Erfindung die Aufgabe zugrunde, ein Instrument zur provisorischen Fixierung der Polyaxialität einer polyaxialen Pedikelschraube zu schaffen, dass die vorstehenden Nachteile nicht aufweist. Insbesondere soll das Instrument einfach handhabbar sein, ein sicheres Ansetzen an der Aufnahmehülse ermöglichen, so dass ein versehentliches Lösen des Instruments von der Pedikelschraube im Wesentlichen nicht möglich ist, eine stabile provisorische Fixierung ermöglichen, so dass eine Manipulation eines Wirbels ermöglicht wird, ein Einsetzen eines Längsträgers in die Aufnahmehülse bei daran angeordnetem Instrument ermöglichen, und ein Einsetzen eines Verriegelungselements, zum Beispiel in Form einer Setscrew oder einer Madenschraube, in die Aufnahmehülse ermöglichen, insbesondere im provisorisch fixierten Zustand. Das Instrument soll einfach und sicher handzuhaben und zu reinigen sowie kostengünstig sein.

Diese Aufgabe wird nach der vorliegenden Erfindung gelöst durch ein Instrument nach dem Oberbegriff von Anspruch 1, wobei das Instrument eine Hülse aufweist, in der die Koppeleinheit in die Axialrichtung bewegbar angeordnet ist, derart, dass die Koppeleinheit in einer ersten Axialposition mit der Aufnahmehülse koppelbar oder entkoppelbar ist, und in einer zweiten Axialposition an der Aufnahmehülse nicht lösbar festgelegt ist.

Neben der Aufgabe eine stabile Verbindung zwischen Tulpe und Instrument herzustellen, ist das Instrument nach der Erfindung insbesondere dazu vorgesehen und geeignet, die Polyaxialität einer polyaxialen Pedikelschraube provisorisch zu fixieren. Es ist mittels der Koppeleinheit an der Aufnahmehülse der Pedikelschraube anzuordnen oder lösbar zu befestigen, um eine provisorische Fixierung, das heißt eine Fixierung die wahlweise eine Kopplung hervorruft oder diese aufhebt, der Pedikelschraube zu realisieren. Die Koppeleinheit kann insbesondere mit einer dazu vorgesehenen Koppelstruktur mit einer an der Aufnahmehülse entsprechend ausgebildeten Gegenkoppelstruktur in Eingriff gebracht werden. Über eine Relativpositionierung von Koppeleinheit und Hülse kann das Instrument einerseits in einen koppelbaren Zustand und andererseits in einen gesicherten oder nicht lösbaren oder unentkoppelbaren Zustand gebracht werden. Ist das Instrument mit der Aufnahmehülse nicht lösbar gekoppelt, kann über den Druckstößel eine Druckkraft auf den in der Aufnahmehülse angeordneten Klemmstempel der Pedikelschraube ausgeübt werden, so dass der Klemmstempel gegenüber der Aufnahmehülse relativpositioniert oder mit der Druckkraft belastet wird, und der Schaftkopf zwischen der Aufnahmehülse und dem Klemmstempel geklemmt und provisorisch lagefixiert wird. Nach der Erfindung können die Koppeleinheit und die Hülse in eine erste Axialposition zueinander positioniert werden. In dieser ist zumindest ein Entkoppeln der Koppeleinheit von der Aufnahmehülse, vorzugsweise ein Entkoppeln von, sowie ein Koppeln mit der Aufnahmehülse der Pedikelschraube möglich. In der zweiten Axialposition von Koppeleinheit und Hülse zueinander ist ein Lösen der Koppeleinheit von der Aufnahmehülse der Pedikelschraube nicht möglich und kann durch eine geeignete Zusammenwirkung von Hülse und Koppeleinheit miteinander verhindert werden, um zusammen mit dem Druckstößel eine provisorische Fixierung der Pedikelschraube zu realisieren. Man kann also sagen, dass die Hülse ein Sicherungselement darstellt, das die Koppeleinheit einerseits (in der ersten Axialposition) für ein Entkoppeln oder ein Koppeln freigibt und andererseits (in der zweiten Axialposition) gegen ein Entkoppeln sperrt.

Vorteilhafte Ausführungsformen der Erfindung sind in den Unteransprüchen beansprucht und werden nachfolgend näher erläutert.

Eine Ausführungsform der Erfindung ist dadurch gekennzeichnet, dass die Koppeleinheit in einem distalen Endbereich eine radial nach innen vorstehende Raststruktur aufweist, zum koppelnden Eingriff in eine dafür vorgesehene Gegenraststruktur der Aufnahmehülse. Die Gegenraststruktur ist in diesem Fall vorzugsweise auf der radial äußeren Seite der Aufnahmehülse ausgebildet, so dass der innerhalb der Aufnahmehülse ausgebildete Aufnahmeraum für einen Längsträger sowie das üblicherweise innerhalb der Aufnahmehülse vorgesehene Innengewinde zu Einschrauben einer Setscrew auch bei gekoppeltem Instrument zugänglich sind. Das bietet den Vorteil, dass Längsträger und/oder Setscrew auch bei provisorischer Fixierung der Polyaxialität der Pedikelschraube manipuliert und gehandhabt werden können. Die Gegenraststruktur an der Aufnahmehülse kann insbesondere in Form eines in Richtung der Schraubenachse radial zurückspringenden Anschlags ausgebildet sein, so dass die Aufnahme schlank ist und bei abgezogenen Instrument keine scharfkantig vorspringenden Teile aufweist.

Alternativ oder zusätzlich kann die Raststruktur der Koppeleinheit in radialer Richtung zur Hülse relativpositionierbar sein, insbesondere infolge von elastischer Verformung der Koppeleinheit in radiale Richtung. Auf diese Weise kann die Raststruktur eine erste Radialposition einnehmen, in der sie in die Gegenraststruktur an der Aufnahmehülse eingreift und das Instrument an dieser fixiert und mit dieser koppelt. Sie kann des Weiteren eine zweite Radialposition einnehmen, in der die Raststruktur außer Eingriff mit der Gegenraststruktur ist und eine Entkopplung oder eine Kopplung erfolgen kann.

Es ist von besonderem Vorteil, wenn die Koppeleinheit mindestens einen sich in die Axialrichtung erstreckenden Koppelarm aufweist, dessen distaler Endbereich in radialer Richtung positionierbar ist, insbesondere infolge von elastischer Verformung in radiale Richtung. Ein solcher Koppelarm baut in vorteilhafter Weise schlank auf, so dass er innerhalb der Hülse nicht viel Bauraum benötigt. Des Weiteren kann ein solcher Arm eine hohe Flexibilität bzw. Elastizität in radialer und/oder tangentialer Richtung aufweisen, bei hoher Steifigkeit in axialer Richtung. Nach einer Ausführungsform kann die Koppeleinheit eine Mehrzahl an Koppelarmen aufweisen. Diese können insbesondere tangential voneinander beabstandet und zueinander symmetrisch ausgebildet oder angeordnet sein. Zum Beispiel kann die Koppeleinheit zwei einander mit Bezug auf die Axialrichtung oder Längsachse gegenüberliegende Koppelarme aufweisen, die tangential voneinander um jeweils 180° beabstandet sind. In ähnlicher Weise kann die Koppeleinheit drei oder vier Koppelarme aufweisen. Ein besonderer Vorteil der Ausführungsform mit zwei Koppelarmen ist, dass zwischen diesen ausreichend Platz vorhanden ist, der zum Durchführen des Längsträgers, der Setscrew und/oder anderen Einheiten wie dem Druckstößel oder einem Stabdrücker zum Drücken des Längsträges in die Aufnahmehülse genutzt werden kann.

Der Koppelarm bzw. die Koppeleinheit kann insbesondere in der Hülse in Axialrichtung bewegbar geführt sein. Dazu kann zum Beispiel in der Hülse eine Führungsnut oder Führungsschulter ausgebildet sein, mit der der Koppelarm bzw. die Koppeleinheit in führender Anlage steht und derart geführt ist.

Bevorzugt ist der Übergang von dem Koppelarm zu der Raststruktur derart ausgebildet ist, dass die radial äußere Seite der Raststruktur gegenüber der radial äußeren Seite des jeweiligen Koppelarms radial nach außen vorsteht.

Eine Ausführungsform der Erfindung ist dadurch gekennzeichnet, dass der distale Endbereich der Koppeleinheit in der zweiten Axialposition, in der die Koppeleinheit nicht lösbar an der Aufnahmehülse festgelegt ist, in radialer Richtung an der Hülse anliegt. Die Hülse bildet in diesem Fall eine Außenumrandung oder Stütze für den distalen Endbereich aus, der sich so nicht mehr in radialer Richtung verformen oder bewegen kann. Ein Lösen der Koppelstruktur von der Gegenkoppelstruktur der Aufnahmehülse wird durch die Anlage an der Hülse verhindert. Nach einer Ausführungsform können die Hülse und der distale Endbereich der Koppeleinheit derart ausgebildet und aufeinander abgestimmt sein, dass die Hülse bei einer Relativbewegung von der ersten Axialposition in die zweite Axialposition eine in einen Eingriff mit der Gegenraststruktur bringende Bewegung der Raststruktur verursacht. Zum Beispiel kann bzw. können die Koppeleinheit und/oder die Hülse mit einer Anlaufschräge oder Führungskontur versehen sein, die bei einer Axialpositionierung von der ersten in die zweite Axialposition eine Zustellbewegung der Koppelstruktur in radialer Richtung bewirkt. Auf diese Weise kann dem Operateur ein Feedback gegeben werden, ob die Koppeleinheit mit der Aufnahmehülse gekoppelt ist und ob diese Kopplung gesichert ist. Lässt sich nämlich die Hülse nicht in die zweite Axialposition bringen, liegt keine Kopplung vor. Es ist besonders vorteilhaft, wenn die Aufnahmehülse der Pedikelschraube in radialer Richtung gesehen außenseitig eine Rampen- bzw. Vorsprungs-Struktur in Axialrichtung für die Koppelstruktur der Koppeleinheit aufweist. Dadurch lässt sich die Koppelstruktur in eine Axialrichtung zum Ineingriffbringen leicht in die Gegenkoppelstruktur der Aufnahmehülse einführen und einrasten, während sie in gegenläufige Axialrichtung durch die Rampen- bzw. den Vorsprungs-Struktur vom einfachen Lösen abgehalten wird. Beispielsweise kann der Operateur durch die Rampenstruktur eine Pedikelschraube mit der Koppeleinheit leicht aufnehmen und koppeln, wohingegen es zum Trennen eines gewissen Mechanismus Bedarfs.

Eine Ausführungsform der Erfindung ist dadurch gekennzeichnet, dass die Koppeleinheit und die Hülse über ein Gewinde miteinander in Eingriff stehen und durch gegenseitiges Verdrehen axialpositionierbar sind. Insbesondere kann eine Gewindehülse oder Gewindemutter vorgesehen sein, die mit einem an der Koppeleinheit ausgebildeten Gegengewinde in Eingriff zu bringen ist oder in Eingriff steht. Die Gewindehülse/Gewindemutter ist drehbar aber axialfest mit der Hülse gekoppelt. Durch Verschrauben der Gewindehülse/Gewindemutter mit dem Gegengewinde erfolgt eine Axialpositionierung von Hülse und Koppeleinheit relativ zueinander. Insbesondere können die Gewindehülse/Gewindemutter mit einem Innengewinde und die Koppeleinheit mit einem Außengewinde als Gegengewinde versehen sein. Es liegt im Bereich der Erfindung, wenn die Hülse mit dem Gegengewinde versehen ist und die Koppeleinheit drehbar aber axialfest mit der Gewindehülse/Gewindemutter gekoppelt ist. Durch die vorstehend beschriebene Ausführungsform wird eine sehr genaue, patientenschonende und nur geringe Betätigungskraft erfordernde Verstellmöglichkeit bewirkt. Das Gewinde kann insbesondere mit einer gewissen Selbsthemmung oder selbsthemmend ausgebildet sein, so dass ein versehentliches Positionieren von Koppeleinheit und Hülse in die zweite Axialposition sicher verhindert werden kann.

Nach einer Ausführungsform der Erfindung ist vorgesehen, dass die Hülse an ihrem distalen Ende eine Aufnahme für einen Abschnitt der Aufnahmehülse aufweist. Dieser kann beispielsweise in Form einer Nut, eines Vorsprungs oder eines Hinterschnitts ausgebildet sein und insbesondere in umfänglicher Richtung ausgebildet sein und abschnittsweise oder vollständig umlaufen. Insbesondere kann die Aufnahme an einer distalen Stirnfläche, die im Wesentlichen quer zur Längsachse ausgerichtet sein kann, ausgebildet sein. Der Abschnitt der Aufnahmehülse und die Aufnahme dafür sind vorzugsweise derart ausgebildet und eingerichtet, dass sie über Formschluss, ggf. Kraftschluss, miteinander koppelbar sind.

Eine weitere Ausführungsform der Erfindung ist dadurch gekennzeichnet, dass die Hülse einen zentralen Durchlass in Axialrichtung aufweist, der quer zur Axialrichtung eine im wesentliche ovale oder elliptische Querschnittsform aufweist, zur um die Axialrichtung drehbaren Aufnahme eines Exzenters zum Aufspreizen der Hülse und/oder der Koppeleinheit.

Es ist von besonderem Vorteil, wenn die Koppeleinheit mit einem zentralen Durchlass in Axialrichtung versehen ist, in der der Druckstößel axialpositionierbar angeordnet ist. Auf diese Weise ist eine direkte und sichere Einwirkung auf den in der Aufnahmehülse gelagerten Klemmstempel sichergestellt. Der Druckstößel kann insbesondere im Wesentlichen hohlzylinderförmig ausgebildet sein und einen zentralen Durchlass in Axialrichtung aufweisen. Er kann des Weiteren ein sich in Axialrichtung erstreckendes Fenster oder Öffnungen aufweisen, die für eine Zugänglichkeit des in der Aufnahmehülse ausgebildeten Innengewindes zum Einschrauben der Setscrew auch bei aufgesetztem Instrument und insbesondere im provisorisch fixierten Zustand der Polyaxialität sorgt, als auch die Möglichkeit bietet, einen Stabdrücker oder weitere Instrumente in Axialrichtung von proximaler Seite des Instruments her einzuführen.

Nach einer Ausführungsform der Erfindung weist der Druckstößel am proximalen Ende eine im Wesentlichen zylinderförmige, insbesondere hohlzylinderförmige, Basis auf, die um die Axialachse drehbar aber axialfest mit dem Druckstößel gekoppelt ist. Diese Basis steht in Eingriff mit der Koppeleinheit und dient der Axialpositionierung des Druckstößels in der Koppeleinheit und damit relativ zur Aufnahmehülse der Pedikelschraube. Vorzugsweise ist das entsprechende Außengewinde an der Basis vorgesehen, über das der Druckstößel und die Koppeleinheit über ein Innengewinde der Koppeleinheit miteinander in Eingriff stehen und durch gegenseitiges Verdrehen axialpositionierbar sind. Vorzugsweise weist die Basis des Druckstößels innenseitig eine Struktur auf, insbesondere in Form einer umlaufenden Nabe, die dafür geeignet ist, ein Drehmoment auf die Basis aufzubringen. Dadurch kann die Axialpositionierung des Druckstößels mittels eines Werkzeugs durchgeführt werden. An der Oberseite des proximalen Endes der Basis ist vorzugsweise umlaufend eine Struktur vorgesehen, insbesondere eine geriffelte Struktur/Riffelstruktur, die dafür geeignet ist, in eine Gegenstruktur formschlüssig einzugreifen und dadurch ebenso ein Drehmoment zu übertragen. Ferner ist es von Vorteil, wenn ein Bereich der Seitenwand des Druckstößels, vorzugsweise entlang der Axialrichtung, eine Öffnung für eine einfach Zugänglichkeit zum Innenraum aufweist.

Eine weitere Ausführungsform der Erfindung ist dadurch gekennzeichnet, dass das Instrument einen Stabdrücker, auch als Rodpusher bezeichnet, aufweist, zum Andrücken eines in der Aufnahmehülse der Pedikelschraube zu fixierenden Stabs/Längsträgers/Spinalstabs in distaler Richtung. Der Stabdrücker kann insbesondere in dem zentralen Durchlass des Druckstößels in Axialrichtung axialpositionierbar sein.

Eine weitere Ausführungsform der Erfindung ist dadurch gekennzeichnet, dass der Stabdrücker ebenfalls einen zentralen Durchlass in Axialrichtung aufweist. Dieser Durchlass kann dazu dienen, eine Setscrew durch das Instrument hindurch, also insbesondere im provisorisch fixierten Zustand der Pedikelschraube, in die Aufnahmehülse einzubringen. Des Weiteren kann ein Schraubwerkzeug zur Schraubbetätigung der Pedikelschraube und/oder zur Schraubbetätigung der Setscrew durchgeführt werden.

In einer Ausführungsform der Erfindung weist der Stabdrücker an seinem proximalen Ende einen frei drehbaren aber axialfest mit dem Stabdrücker gekoppelten und im Wesentlichen zylinderförmigen, insbesondere hohlzylinderförmigen, geformten Schraubkopf auf. Dieser Schraubkopf dient der Einstellung der Axialpositionierung des Stabdrückers in der Koppeleinheit und damit der Axialpositionierung zum Stab. Das distale Ende des Schraubkopfs ist vorzugsweise mit einem Außengewinde versehen. Dieses Außengewinde greift in das entsprechend vorgesehene Innengewinde der Koppeleinheit, wobei durch Verdrehen die Axialposition des Stabdrückers eingestellt werden kann. Vorzugsweise ist die Unterseite des distalen Endes des Schraubkopfs mit einer Struktur versehen, insbesondere einer Riffelstruktur, die dafür geeignet ist in eine zweite Struktur, insbesondere der Riffelstruktur des Druckstößels, einzugreifen, und so formschlüssig ein Drehmoment zu übertragen. Vorzugsweise ist an der radialen Außenseite des Schraubkopfs eine Skalierung vorgesehen, an welcher der Operateur eine axiale Positionierung bzw. Einschraubtiefe ablesen kann. Es ist besonders vorteilhaft, wenn am proximalen Ende des Schraubkopfes, insbesondere innseitig, eine Struktur vorgesehen ist, die dafür geeignet ist, ein Drehmoment zu übertragen. In diese Struktur kann ein Werkzeug formschlüssig eingesetzt werden und so der Schraubkopf über ein entsprechend vorgesehenes Außengewinde in das Innengewinde der Koppeleinheit eingeschraubt und axialpositioniert werden. Durch einen zentralen Durchlass in Axialrichtung kann ein Instrument, insbesondere ein Schraubwerkzeug, eingeführt werden. Ferner ist, vorzugsweise an der Innenseite des Schraubkopfs in der Nähe des proximalen Endes, eine Struktur vorgesehen, insbesondere ein Innengewinde, über das ein Schraubwerkzeug eingeschraubt werden kann, und in der Nähe des distalen Endes eine geometrische Begrenzung vorgesehen, wodurch nach Einsetzen eines Instruments, insbesondere eines Schraubwerkzeugs, dieses zwischen zwei Positionen innerhalb des Stabdrückers in Axialrichtung bewegbar angeordnet ist.

In einer Ausführungsform der Erfindung umfasst das medizintechnische Instrument ferner ein Schraubwerkzeug. Dieses Schraubwerkzeug weist am distalen Ende eine Struktur/Mitnahmeprofil auf, die dafür geeignet ist, formschlüssig ein Drehmoment bzw. Schraubbetätigung auf insbesondere die Setscrew und/oder die Pedikelschraube aufzubringen. Ferner weist das Schraubwerkzeug am proximalen Ende eine Struktur/Aufnahmeprofil auf, die dafür geeignet ist, formschlüssig ein Drehmoment aufzunehmen, insbesondere durch ein Griffstück. Das Schraubwerkzeug ist in Axialrichtung gesehen in einem mittleren Bereich mit einer Struktur versehen, vorzugsweise mit einem Außengewinde, welche dafür geeignet ist, in die oben beschriebene (Gegen-)Struktur des Schraubkopfes, insbesondere das Innengewinde, einzugreifen, wodurch sich das Schraubwerkzeug beispielsweise gegen ein versehentliches Herausfallen sichern lässt.

In einer weiteren Ausführungsform der Erfindung weist das medizinische Instrument einen Stabdrücker auf, zum Andrücken eines in der Aufnahmehülse der Pedikelschraube zu fixierenden Stabs oder Längsträgers in distaler Richtung, wobei der Druckstößel und der Stabdrücker als einteilige Druckeinheit ausgebildet sind. Die Druckeinheit weist in ihren Ausführungsformen daher vorzugsweise weiterhin die oben beschriebenen Merkmale von Druckstößel und Stabdrücker auf, insbesondere einen Führungsvorsprung zum Führen der Druckeinheit in der Hülse, einen zentralen Durchlass in Axialrichtung, einen Schraubkopf sowie Stützzapfen am distalen Ende.

Durch Axialpositionierung der einteiligen Druckeinheit in der Hülse erfolgt sowohl eine provisorische Fixierung der Polyaxialität der Pedikelschraube als auch eine Lagefixierung des Stabs. Es kann ein Schraubwerkzeug zur Schraubbetätigung der Pedikelschraube und/oder zur Schraubbetätigung der Setscrew zusammen mit einer Setscrew vom proximalen Ende des Instruments her durch einen zentralen Durchlass der Druckeinheit hindurchgeführt werden. Durch die einteilige Ausführung von Druckstößel und Stabdrücker lässt sich unter anderem der zentrale Durchlass größer gestalten.

In einer bevorzugten Ausführungsform zeichnet sich das medizinische Instrument dadurch aus, dass die Raststruktur derart in eine von der Hülse ausgeformte Gegenraststruktur eingreift, dass von der Koppeleinheit bei einer entsprechenden Betätigung durch einen Operateur eine Zugkraft, das heißt eine Kraft in proximaler Richtung, auf die Hülse übertragbar ist. Somit ist das Instrument in der Lage, Korrekturen in der Fixierung der Pedikelschraube vorzunehmen, was ein effizientes Anbringen der Schraube im Patienten erleichtert.

Insbesondere vorteilhaft ist es hierbei, wenn die Gegenraststruktur zumindest eine Fläche aufweist, die zumindest teilweise in horizontaler Richtung verläuft und zu der distal die Raststruktur anordenbar ist, um die Zugkraft formschlüssig zu übertragen. Jene Fläche verläuft bevorzugt zumindest teilweise, in der Ebene, auf welcher die Längsachse des Instruments orthogonal steht. So wird es der Raststruktur ermöglicht, bei einer Zugbewegung, das heißt bei einer Bewegung in Richtung distal, einen Anschlag in der Gegenraststruktur zu kontaktieren, was eine zuverlässige Kraftübertragung garantiert.

Sobald die Gegenraststruktur in ihrem distalen Endbereich, das heißt an dem dem Schaft zugewandten Abschnitt, eine bauchige / teilkreisförmige / tropfenförmige Form aufweist, lässt sich der Formschluss zur Zugkraftübertragung effizient realisieren. Jene Geometrie bezieht sich hierbei auf die Form der Gegenraststruktur in einer Frontalansicht. Überdies oder alternativ ist es von Vorteil, wenn die Gegenraststruktur zwei entgegengesetzte, in Umfangsrichtung herausstehende Ausnehmungen in Form einer Hinterschneidung / eines Hinterschnitts aufweist, sodass die Gegenraststruktur etwa eine kreuzförmige Gestalt annimmt.

Zusammenfassend kann man sagen, dass die Erfindung ein Instrument bereitstellt, das auch als Downtube bezeichnet wird, mit einer Verriegelungsmöglichkeit der Polyaxialklemmung und einer Verriegelungsfunktion des Implantats.

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung.

### Kurzbeschreibung Figuren

Fig. 1 bis Fig. 16 zeigen ein medizintechnisches Instrument gemäß einer ersten Ausführungsform der vorliegenden Erfindung. Fig. 17 bis Fig. 20 zeigen ein Instrument gemäß einer zweiten Ausführungsform der vorliegenden Erfindung.

Es zeigen im Einzelnen:
Fig. 1 in einer perspektivischen Darstellung ein Koppelelement eines Instruments nach der Erfindung,
Fig. 2 in einer perspektivischen Darstellung eine Hülse eines Instruments nach der Erfindung,
Fig. 3 in einer perspektivischen Darstellung einen Druckstößel eines Instruments nach der Erfindung,
Fig. 4 in einer perspektivischen Darstellung einen Stabdrücker eines Instruments nach der Erfindung,
Fig. 5 eine Koppeleinheit und eine Hülse zusammengesteckt in einer perspektivischen Darstellung in einer ersten Axialposition,
Fig. 6 die Koppeleinheit und die Hülse der Figur 5 mit einer polyaxialen Pedikelschraube in der ersten Axialposition,
Fig. 7 die Koppeleinheit und die Hülse der Figuren 5 und 6 mit der polyaxialen Pedikelschraube in der zweiten Axialposition,
Fig. 8 einen Querschnitt durch die Hülse
Fig. 9 einen Querschnitt durch die Hülse mit Pedikelschraube mit Druckstempel und Setscrew,
Fig. 10 in einer ausschnittsweisen perspektivischen Darstellung den Übergang von der ersten Axialposition in die zweite Axialposition,
Fig. 11A eine perspektivische Darstellung eines vergrößerten Ausschnitts des Verbindungsbereichs der Pedikelschraube,
Fig. 11B eine perspektivische Darstellung eines vergrößerten Ausschnitts des Verbindungsbereichs von Instrument und Pedikelschraube,
Fig. 12 eine perspektivische Darstellung eines vergrößerten Ausschnitts des Verbindungsbereichs von Instrument und Pedikelschraube mit eingeführtem Druckstößel mit und ohne Längsträger,
Fig. 13 eine seitliche Ansicht eines vergrößerten Ausschnitts des Verbindungsbereichs von Instrument und Pedikelschraube mit eingeführtem Druckstößel,
Fig. 14 in vergrößerter perspektivischer Ansicht das Einpressen eines Längsträgers durch den Stabdrücker in die Aufnahmehülse,
Fig. 15 in vergrößerter perspektivischer Ansicht das Einschrauben einer Setscrew in die Aufnahmehülse,
Fig. 16 in einer perspektivischen Darstellung ein Schraubwerkzeug eines Instruments nach der Erfindung.
Fig. 17 in einer perspektivischen Darstellung eine Druckeinheit nach einer zweiten Ausführungsform eines Instruments nach der Erfindung,
Fig. 18 eine perspektivische Darstellung eines vergrößerten Ausschnitts des Verbindungsbereichs von Instrument und Pedikelschraube mit eingeführter Druckeinheit mit und ohne Längsträger nach einer zweiten Ausführungsform,
Fig. 19 in vergrößerter perspektivischer Ansicht das Einpressen eines Längsträgers durch die Druckeinheit in die Aufnahmehülse nach einer zweiten Ausführungsform, und
Fig. 20 in vergrößerter perspektivischer Ansicht das Einschrauben einer Setscrew in die Aufnahmehülse nach einer zweiten Ausführungsform,

### Detaillierte Beschreibung

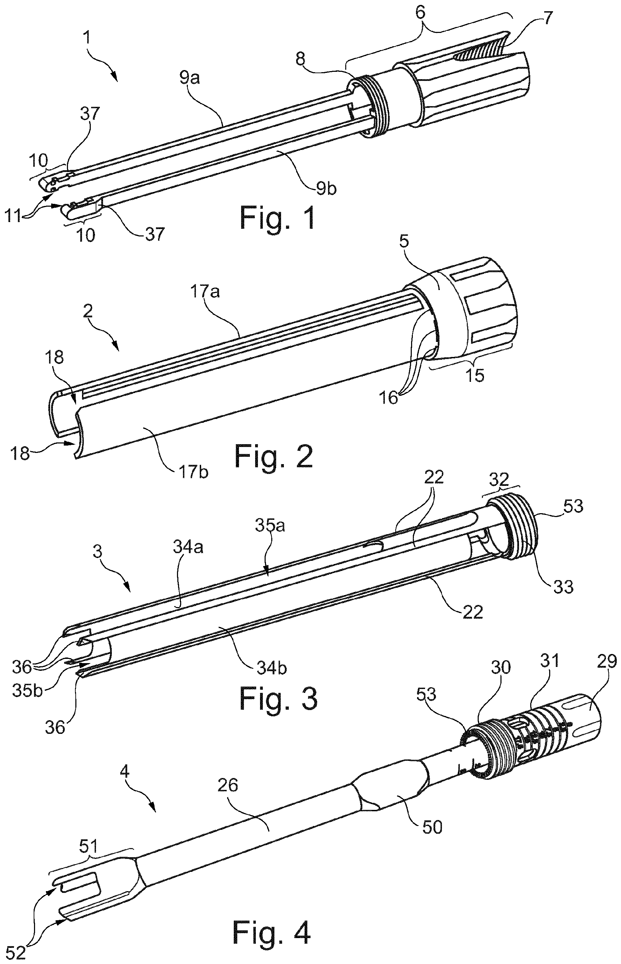

Ein medizintechnisches Instrument einer ersten Ausführungsform nach der Erfindung umfasst eine Koppeleinheit 1 (die auch als Koppelement 1 bezeichnet werden kann), eine Hülse 2, einen Druckstößel 3, einen Stabdrücker 4 und eine Stellmutter 5. Ferner kann das medizintechnische Instrument nach der Erfindung ein Schraubwerkzeug 45 umfassen. Figur 1 zeigt die Koppeleinheit 1 bzw. das Koppelelement 1, Figur 2 die Hülse 2 mit der daran drehbar aber in zumindest eine Richtung axialfesten Stellmutter 5, Figur 3 den Druckstößel 3 und Figur 4 den Stabdrücker 4, jeweils in einer perspektivischen Darstellung. In den Figuren 1 bis 7 sowie 16 und 17 stellt jeweils die linke Seite der Figur die distale Seite und jeweils die rechte Seite der Figur die proximale Seite des gezeigten Elements bzw. des Instruments dar. Die Längsachse oder Axialrichtung im Sinne der vorliegenden Beschreibung erstreckt sich von der distalen Seite zur proximalen Seite und umgekehrt.

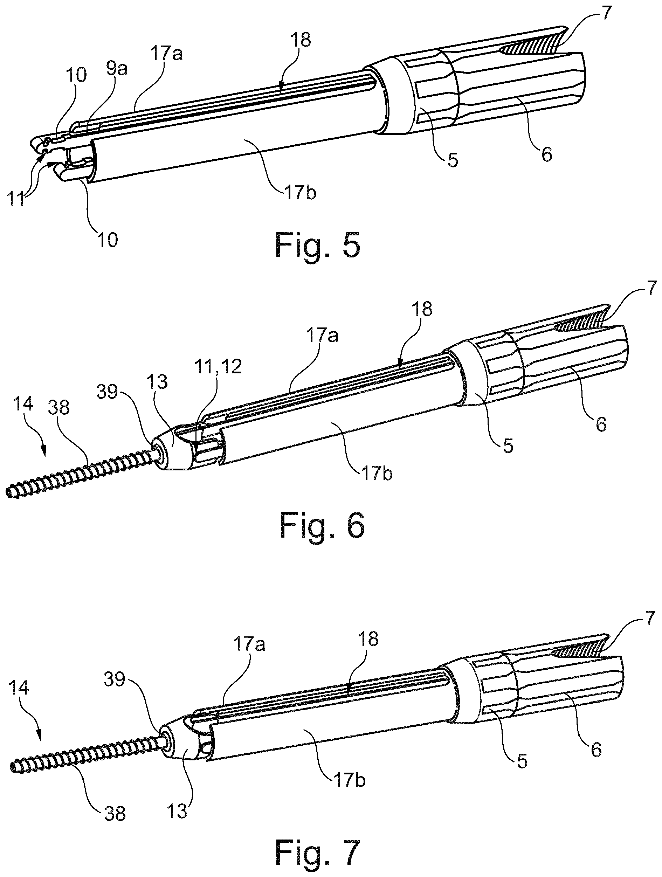

Das Koppelelement 1 weist eine im Wesentlichen hohlzylinderförmige proximale Basis 6 auf, die mit einem Innengewinde 7 und an ihrem distalen Endbereich mit einem Außengewinde 8 versehen ist. Von der Basis 6 aus erstrecken sich zwei Koppelarme 9a, 9b in distale Richtung. Die Koppelarme 9a, 9b sind zueinander parallel und liegen einander diametral gegenüber, d.h. sie schließen zwischen sich zu beiden Seiten jeweils einen Winkel von 180° ein. Am distalen Endabschnitt jedes Koppelarms 9a, 9b ist jeweils eine Koppelstruktur 10 ausgebildet. Auf der radial äußeren Seite ist der Übergang vom Koppelarm 9a, 9b zur Koppelstruktur 10 jeweils mit einer Schräge 37 ausgebildet, und die radial äußere Seite der Koppelstruktur 10 springt gegenüber der radial äußeren Seite des jeweiligen Koppelarms 9a, 9b nach radial außen vor. Auf der radial inneren Seite jeder Koppelstruktur 10 ist eine Raststruktur 11 ausgebildet, zum Eingriff in eine entsprechende Gegenraststruktur 12, die an einer Aufnahmehülse 13 einer polyaxialen Pedikelschraube 14 ausgebildet ist (siehe zum Beispiel in Figur 6, 9 oder 10). Die Raststruktur 11 und die Gegenraststruktur 12 sind jeweils derart ausgebildet, dass sie sowohl in axialer Richtung als auch in tangentialer Richtung (umfänglich) verrasten.

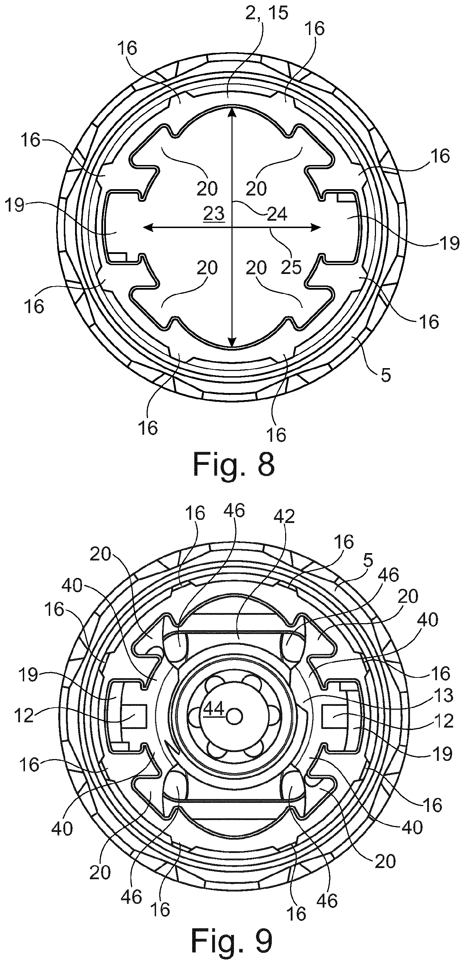

Die Hülse 2 weist ebenfalls eine proximale Basis 15 auf, deren distaler Endbereich mit einer Mehrzahl von Schultern 16 oder Anschlägen 16 ausgebildet ist. Diese dienen als Anlage für die Stellmutter 5, so dass sich die Hülse 2 nicht in die distale Richtung aus der der Stellmutter 5 herausbewegen kann, bzw. die Stellmutter 5 nicht proximal von der Hülse 2 entfernt werden kann. Distal der Basis 15 weist die Hülse zwei einander diametral gegenüber liegend angeordnete teilzylinderförmige Hülsenarme 17a, 17b auf. Jeder Hülsenarm 17a, 17b erstreckt sich in umfänglicher Richtung um ca. 90°, so dass zwischen ihnen zwei einander diametral gegenüber liegende Fenster 18 oder Zwischenräume 18 ausgebildet sind. In den Figuren 8 und 9 ist die Hülse 2 in einem Querschnitt quer zur Axialrichtung in Höhe ihrer proximalen Basis 15 gezeigt. Deutlich zu erkennen sind die radial nach außen vorragenden Schultern/Anschläge 16. Radial nach Innen ist die Hülse 2 mit zwei einander gegenüber liegenden Führungsnuten 19 für die Koppelarme 9a bzw. 9b ausgebildet. Beiderseits jeder Führungsnut 19 ist jeweils eine Führungsnut 20, also insgesamt vier Führungsnuten 20, ausgebildet, zur führenden Aufnahme jeweils eines Führungsvorsprungs 22 des Druckstößels 3. Die Figuren 8 und 9 zeigen auch deutlich, dass die Hülse 2 von einem durchgehenden Durchlass 23 durchdrungen ist, der in Axialrichtung ausgebildet ist. Dieser Durchlass 23 ist im Querschnitt oval oder elliptisch geformt. Seine große Halbachse 24 erstreckt sich von einem Fenster 18 zum gegenüber liegenden Fenster 18. Seine kleine Halbachse 25 erstreckt sich quer zur großen Halbachse 24, zum Beispiel von einem Hülsenarm 17a zum gegenüber liegenden Hülsenarm 17b. Diese Ausbildung des Durchlasses 23 dient der Aufnahme eines und dem Zusammenwirken mit einem in den Figuren nicht dargestellten Exzenterinstruments, durch das durch Drehen um die Längsachse die beiden gegenüber liegenden Hülsenarme 17a, 17b gespreizt werden können (Im Rahmen eines Ablösens des Instruments von einer Aufnahmehülse 13 einer Pedikelschraube).

Der Druckstößel 3 ist im Wesentlichen hohlzylinderförmig ausgebildet. Er weist eine proximale Basis 32 auf, die drehbar aber axialfest mit dem Druckstößel 3 gekoppelt ist und die mit einem Außengewinde 33 versehen ist. Distal der Basis 32 weist der Druckstößel 3 zwei einander diametral gegenüber liegend angeordnete teilzylinderförmige Stößelarme 34a, 34b auf. Jeder Stößelarm 34a, 34b erstreckt sich in umfänglicher Richtung um ca. 90°, so dass zwischen ihnen zwei einander diametral gegenüber liegende Fenster 35a, 35 b oder Zwischenräume 35a, 35b ausgebildet sind. Das Fenster 35a ist gegenüber dem Fenster 35b größer ausgebildet, wodurch ein besserer Zugriff in den Innenraum des Druckstößels 3 möglich ist. Das distale Ende des Druckstößels 3 ist mit vier in axialer Richtung vorspringenden Stützzapfen 36 versehen. Das Außengewinde 33 der drehbaren Basis 32 ist zum Eingriff mit dem Innengewinde 7 des Koppelelements 1 bestimmt und ausgebildet. Durch Ein- bzw. Ausschrauben des Druckstößels 3 in das oder aus dem Koppelelement 1 erfolgt deren relative axiale Positionierung zueinander, und damit eine entsprechende axiale Positionierung der Stützzapfen 36 relativ zur Koppelstruktur 10. Der Druckstößel 3 weist des Weiteren die vier gleichförmig umfänglich voneinander beabstandete Führungsvorsprünge 22 auf. Diese sind zum führenden Zusammenwirken mit den Führungsnuten 20 der Hülse 2 bestimmt und ausgebildet.

Der Stabdrücker 4, der in den zentralen Durchlass des Druckstößels 3 eingeführt werden kann, umfasst einen im Wesentlichen zylinderförmigen Stab 26, dessen distaler Abschnitt 51 mit einer Stabaufnahme 52 ausgebildet ist. Das proximale Ende des Stabdrückers 4 weist einen drehbaren aber axialfest mit der Stabdrücker 4 gekoppelten Schraubkopf 29 auf, der mit einem Außengewinde 30 ausgebildet ist und eine Skalierung 31 trägt, über die der Operateur die Einschraubtiefe des Stabdrückers 4 erkennen kann. Das Außengewinde 30 des Schraubkopfs 29 ist zum Eingriff mit dem Innengewinde 7 des Koppelelements 1 bestimmt und ausgebildet. Durch Ein- bzw. Ausschrauben des Stabdrückers 4 in das oder aus dem Koppelelement 1 erfolgt deren relative axiale Positionierung zueinander, und damit eine entsprechende axiale Positionierung der Stabaufnahme 52 relativ zur Koppelstruktur 10. Der Schraubkopf 29 weist an der distalen Unterseite eine geriffelte Struktur 53 auf, die dazu geeignet ist, in die geriffelte Struktur 53 an der proximalen Oberseite der Basis 32 des Druckstößels 3 einzugreifen. In einem mittleren Abschnitt ist eine ovale Form 50 ausgebildet, die dafür geeignet ist, den Stabdrücker 4 in dem Druckstößel 3 in Axialrichtung zu führen und den rotatorischen Freiheitsgrad des zylinderförmigen Stabes 26 des Stabdrückers 4 in Bezug auf den Druckstößel 3 einzuschränken. Am Stabdrücker 4 ist am proximalen Ende an der Innenseite des Schraubkopfes 29 umlaufend eine Nabenstruktur vorgesehen, in die ein nicht dargestelltes Werkzeug formschlüssig eingreifen und ein Drehmoment übertragen kann. Des Weiteren ist an der Innenseite des Schraubkopfs 29 in einem mittleren Abschnitt des Schraubenkopfs 29 in der Nähe des proximalen Endes ein Innengewinde und in der Nähe des distalen Endes eine Begrenzung in Form eines geometrischen Anstoßes vorgesehen. In dieses wird das Außengewinde 49 des Schraubwerkzeugs 45 eingeschraubt. Hiernach ist das Schraubwerkzeug 45 zwischen den zwei Positionen, entsprechend dem Abstand von Innengewinde zu Begrenzung, axialbeweglich.

Die Figuren 5 und 6 zeigen die Koppeleinheit 1 und die Hülse 2 zusammengesteckt in der ersten Axialposition, wobei in Figur 6 eine mittels der Koppelarme 9a, 9b ergriffene Pedikelschraube 14 dargestellt ist. Deutlich zu erkennen ist in Figur 6 ein Gewindeschaft 38, an dessen proximalen Ende ein Schaftkopf 39 ausgebildet ist, auf dem wiederum die Aufnahmehülse 13 dreh- und/oder schwenkbar angeordnet ist. In der in den Figuren 5 und 6 dargestellten ersten Axialposition ragen die Koppelstrukturen 10 jedes Koppelarms 9a, 9b in axial distale Richtung aus der Hülse 2 heraus und stehen gegenüber den distalen Enden der Hülsenarme 17a, 17b vor.

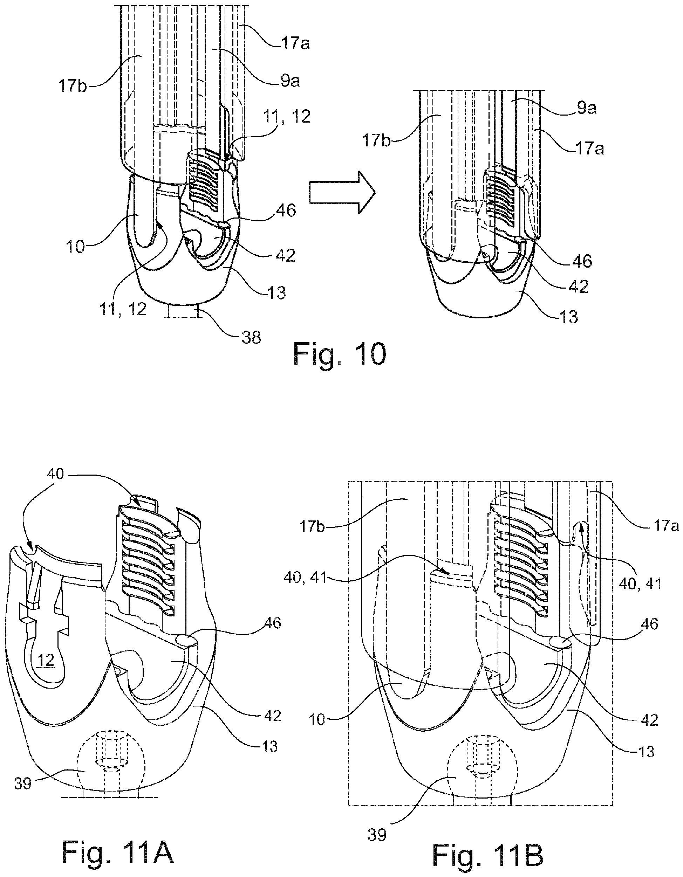

Figur 7 zeigt die Koppeleinheit 1 und die Hülse 2 zusammengesteckt in der zweiten Axialposition mit der gekoppelten Pedikelschraube 14. Durch Verschrauben der Stellmutter 5 ist die Koppeleinheit 1 relativ zur Hülse 2 in die axial proximale Richtung zurückgezogen. Dadurch wurden die Koppelarme 9a, 9b mit den Koppelstrukturen 10 und der damit gekoppelten Aufnahmehülse 13 ebenfalls in die Hülse 2 hineingezogen. Da die radial äußere Seite im Übergang vom Koppelarm 9a, 9b zur Koppelstruktur 10 jeweils mit einer Schräge 37 ausgebildet ist und die radial äußere Seite der Koppelstruktur 10 gegenüber der radial äußeren Seite des jeweiligen Koppelarms 9a, 9b nach radial außen vorspringt und an der Innenseite der Hülsenarme 17a, 17b anliegt, werden die Koppelarme 9a, 9b bei einer Axialbewegung aus der ersten Axialposition in die zweite Axialposition durch Zusammenwirken mit den Hülsenarmen 17a, 17b nach radial innen in einen Eingriff mit der Gegenraststruktur 12 der Aufnahmehülse 13 der Pedikelschraube 14 gepresst. In der in Figur 6 gezeigten zweiten Axialposition ist ein Lösen der Raststruktur 11 der Koppelstrukturen 10 von den jeweiligen Gegenraststrukturen 12 der Aufnahmehülse 13 infolge der Anlage an den Hülsenarmen 17a, 17b nicht möglich. Figur 10 zeigt die Bewegung von der ersten Axialposition (links) in die zweite Axialposition (rechts) in einer vergrößerten Darstellung. In dieser Ausführungsform ist es von Vorteil, dass sowohl die beiden Koppelarme 9a, 9b der Koppeleinheit 1 am distalen Ende an ihrer radialen Innenseite eine leichte Einführschräge aufweisen, als auch die Aufnahmehülse 13 an ihrer radialen Außenseite gegenüberliegend je eine entsprechende Rampenstruktur besitzt, die ein Ineingriffbringen/Koppeln der Raststruktur 11 der Koppelstruktur 10 mit der Gegenraststruktur 12 der Aufnahmehülse 13 erleichtern.

Figur 11A zeigt einen Ausschnitt der Pedikelschraube 14 mit Gegenraststruktur 12 und Rampenstruktur der Aufnahmehülse 13. Figur 11B zeigt deutlich den Eingriff der Hülsenarme 17a, 17b mit der Aufnahmehülse 13 im Detail. In die proximale Stirnseite der Aufnahmehülse 13 ist eine in umfängliche Richtung verlaufende Nut 40 oder ein Hinterschnitt 40 eingebracht. In diese greift eine entsprechend ausgebildete Gegenkontur 41 oder ein Hinterschnitt 41 der Hülsenarme 17a, 17b ein, so dass die Hülse 2 durch Formschluss über die Hinterschnitte 40 und 41 mit der Aufnahmehülse 13 verbunden ist.

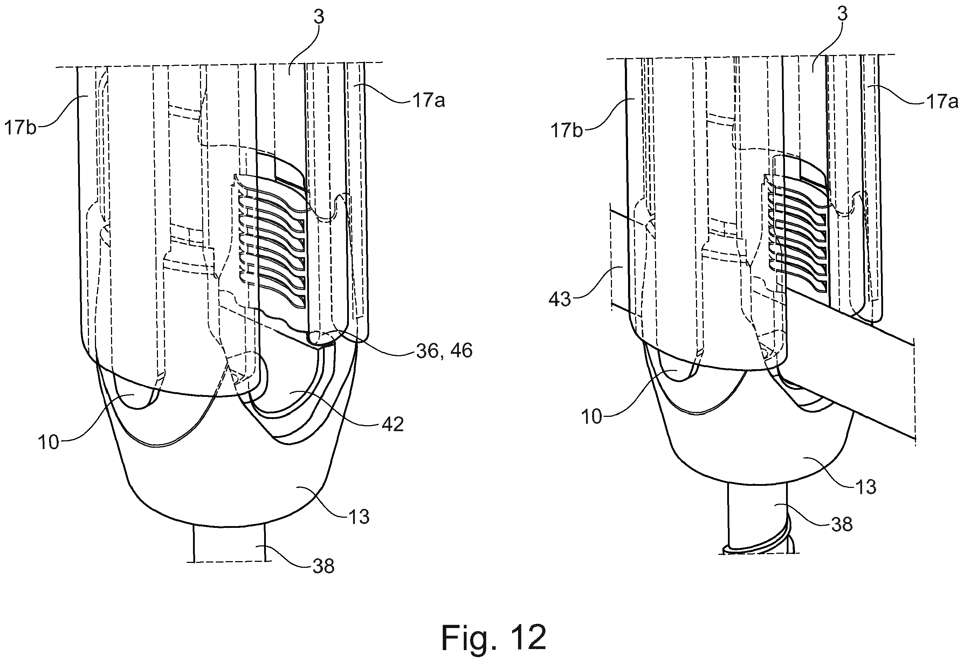

In Figur 12 ist die Funktionsweise des Instruments mit dem Druckstößel 3 dargestellt. Zu erkennen ist insbesondere ein in der Aufnahmehülse 13 gelagerter Klemmstempel 42 mit vier Vertiefungen 46 zum Zusammenwirken und Aufnehmen der Stützzapfen 36. Der Klemmstempel 42 steht mit dem Schaftkopf 39 in Anlage. In beiden Figuren befinden sich die Koppeleinheit 1 und die Hülse 2 in der zweiten Axialposition zueinander, so dass die Aufnahmehülse 13 sicher, axialfest, drehfest und nicht lösbar mit dem Instrument gekoppelt ist. Der Druckstößel 3 ist in den Durchlass 23 eingesetzt. Durch Verschrauben des Druckstößels 3 in dem Innengewinde 7 der Koppeleinheit 1 erfolgt eine entsprechende Positionierung relativ zur Hülse 2 sowie zur Koppeleinheit 1 und damit relativ zu gekoppelten Aufnahmehülse 13 der Pedikelschraube 14. Infolgedessen kommt es zu einer Verspannung des Klemmstempels 42 gegenüber der Aufnahmehülse 13, so dass der Schaftkopf 39 zwischen diese festgespannt wird und die Polyaxialität der Pedikelschraube 14 provisorisch fixiert oder blockiert ist.

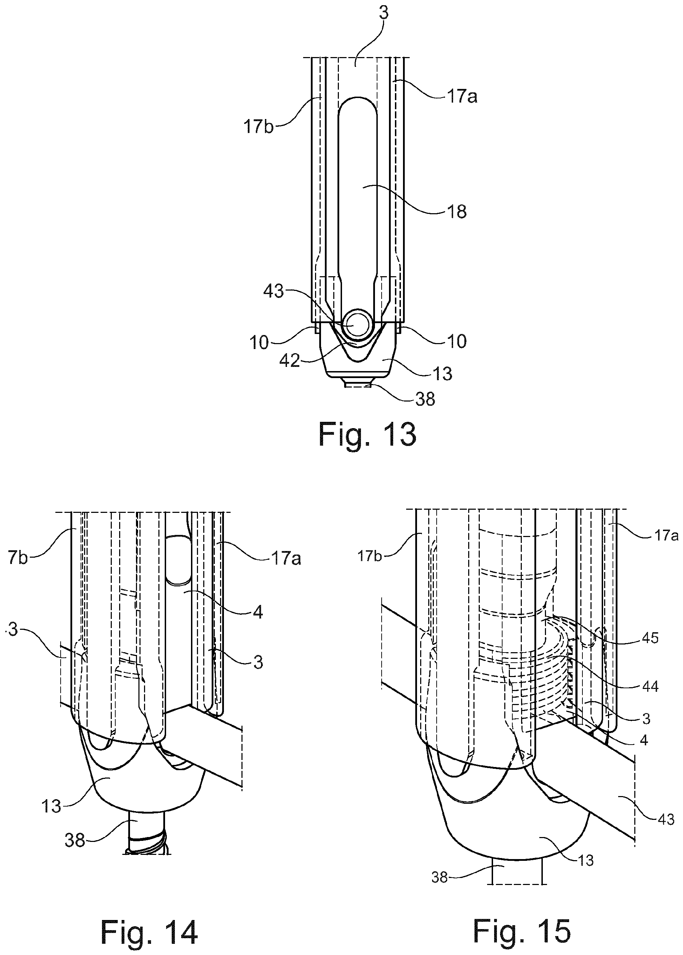

Rechts in der Figur 12 ist ein in der Aufnahmehülse 13 aufgenommener Längsträger 43 gezeigt. Figur 12 zeigt, dass der Druckstößel 3 zwar durch Anlage an dem Klemmstempel 42 die Polyaxialität der Pedikelschraube 14 blockiert, der Längsträger 43 aber in den Fenstern 18 und 35a, 35b relativ frei positionierbar ist (siehe auch Figur 13).

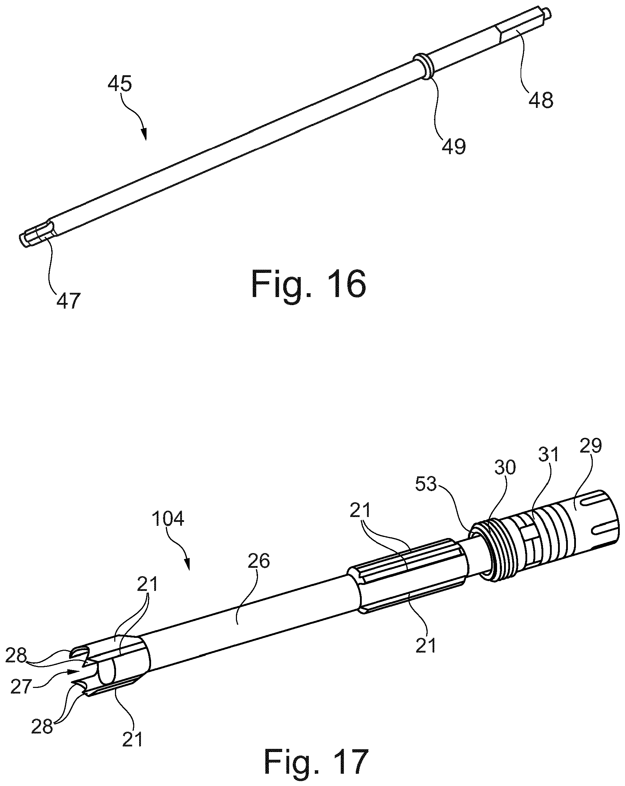

Figur 16 zeigt eine Ausführungsform eines im Wesentlichen zylinderförmigen Schraubwerkzeugs 45 mit einem proximalen Sechskant-Aufsatzprofil 48, um formschlüssig von einem Griff (nicht dargestellt) gegriffen zu werden, einem distalen Mitnahmeprofil 47, um formschlüssig die Setscrew 44 aufzunehmen, sowie einem Außengewinde 49 im mittleren Bereich, um in das Innengewinde des Stabdrückers 4 eingeschraubt zu werden. Das Mitnahmeprofil 47 weist darüber hinaus am distalen Ende einen geschlitzten Ring auf, der mit einem gewissen Spiel zusammendrückbar ist, um eine Setscrew 44 mit einer entsprechenden radial umlaufenden Nut in der Innenseite gegen ein unabsichtliches Lösen von dem Schraubwerkzeug 45 zu sichern.

In Figur 15 ist das Einschrauben einer Setscrew 44 als Verriegelungselement 44 mittels eines Schraubwerkzeugs 45 gezeigt. Die Setscrew 44 kann zusammen mit dem Schraubwerkzeug 45 und dem Stabdrücker 4 durch den Druckstößel 3 hindurch zur Aufnahmehülse 13 geführt und in deren Innengewinde eingeschraubt werden. Dadurch wird der Stab in die Aufnahmehülse 13 eingepresst und die Polyaxialität der Pedikelschraube wird über das Zusammenwirken von Koppeleinheit 1, Hülse 2 und Druckstößel 3 blockiert. Bei der ersten Ausführungsform der vorliegenden Erfindung mit Druckstößel 3 und Stabdrücker 4 wird also zunächst das Schraubwerkzeug 45 durch den Stabdrücker 4 hindurch geführt und an dessen distalem Ende die Setscrew 44 aufgesetzt. Hiernach wird der der Stabdrücker 4 inklusive Schraubwerkzeug 45 und Setscrew 44 durch die vorgesehene Öffnung des Druckstößels 3 hindurch zur Aufnahmehülse 13 geführt und die Setscrew 44 in das Innengewinde der Aufnahmehülse 13 eingeschraubt.

Durch die Teilung von Druckstößel 3 und Stabdrücker 4 kann so in einem ersten Schritt, durch Eindrehen des Druckstößels 3 in das Innengewinde 7 der Koppeleinheit 1, eine provisorische Fixierung der Pedikelschraube 14 erreicht werden, während der Längsträger 43 weiterhin noch beweglich und justierbar ist. Erst in einem zweiten Schritt wird der Stabdrücker 4 in das Innengewinde der Koppeleinheit 1 eingeschraubt, wobei der Längsträger 43 so weit in die Aufnahmehülse 13 gedrückt wird, dass die Setscrew 44 in die Aufnahmehülse 13 einschraubbar wird. In einem dritten Schritt kann nun das Schraubwerkzeug 45 die Setscrew 44 in das Innengewinde der Aufnahmehülse 13 der Pedikelschraube 14 eindrehen, wodurch die Polyaxialität durch den Einschraubdruck der Setscrew 44 auf Stab 43, und weiter auf Stempel und Schaftkopf, dauerhaft blockiert bzw. fixiert wird. Hiernach kann die provisorische Fixierung des medizinischen Instruments gelöst und das medizinische Instrument im entkoppelbaren Zustand von der fixierten von Pedikelschraube 14 getrennt werden.

In Figur 17 ist eine zweite Ausführungsform der vorliegenden Erfindung mit einer einteiligen Ausbildung von Druckstößel 3 und Stabdrücker 4 als Druckeinheit 104 gezeigt. Die Druckeinheit 104 umfasst einen im Wesentlichen zylinderförmigen Stab 26, dessen distales Ende zu einer Stabaufnahme 27 ausgebildet ist und vier in axialer Richtung vorspringende Stützzapfen 28 aufweist. Das proximale Ende der Druckeinheit 104 weist einen drehbaren aber axialfest mit der Druckeinheit 104 gekoppelten Schraubkopf 29 auf, der mit einem Außengewinde 30 ausgebildet ist und eine Skalierung 31 trägt. Das Außengewinde 30 ist, wie auch schon beim Stabdrücker 4, zum Eingriff mit dem Innengewinde 7 des Koppelelements 1 bestimmt und ausgebildet. Durch Ein- bzw. Ausschrauben der Druckeinheit 104 in die oder aus der Koppeleinheit 1 heraus erfolgt, wie bereits beim Stabdrücker 4, deren relative axiale Positionierung zueinander, und damit eine entsprechende axiale Positionierung der Stabaufnahme 27 relativ zur Koppelstruktur 10. In einem mittleren Abschnitt und im distalen Endbereich ist die Druckeinheit 104 mit vier gleichförmig umfänglich voneinander beabstandeten Führungsvorsprüngen 21 versehen. Diese sind zum führenden Zusammenwirken mit den Führungsnuten 20 der Hülse 2 bestimmt und ausgebildet. Der Schraubkopf 29 weist an der distalen Unterseite wieder eine geriffelte Struktur 53 auf.

Unter Verwendung der Druckeinheit 104 kann der Operateur aufgrund der einteiligen Ausführung in einem einzelnen Schritt sowohl die Fixierung der Polyaxialität der Pedikelschraube 14 als auch die Stabdrückfunktion des Längsträgers 43 erreichen.

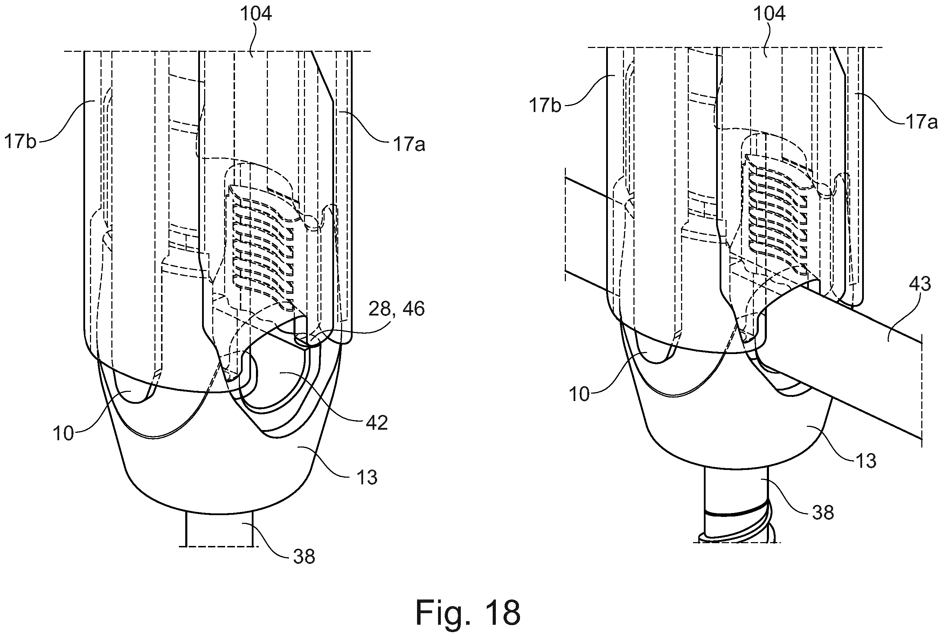

In Figur 18 ist der Verbindungsbereich von Instrument und Pedikelschraube 14 mit eingeführter Druckeinheit 104 der zweiten Ausführungsform der Erfindung gezeigt. Ähnlich der Figur 12 der zweiteiligen Ausführungsform, sowie deren Funktionsweise, ist wieder ein in der Aufnahmehülse 13 gelagerter Klemmstempel 42 mit vier Vertiefungen 46 zum Zusammenwirken und Aufnehmen der Stützzapfen 28 zu erkennen. Durch Verschrauben der Druckeinheit 104 in dem Innengewinde 7 der Koppeleinheit 1 erfolgt eine entsprechende Positionierung relativ zur Hülse 2 sowie zur Koppeleinheit 1 und damit relativ zu gekoppelten Aufnahmehülse 13 der Pedikelschraube 14. Infolgedessen kommt es zu einer Verspannung des Klemmstempels 42 gegenüber der Aufnahmehülse 13, so dass der Schaftkopf 39 zwischen diese festgespannt wird und die Polyaxialität der Pedikelschraube 14 provisorisch fixiert oder blockiert ist. Rechts in der Figur 18 ist ein in der Aufnahmehülse 13 aufgenommener Längsträger 43 gezeigt. Es erfolgt, aufgrund der einteiligen Ausbildung, durch Verschrauben der Druckeinheit 104 in dem Innengewinde 7 ebenso ein Herunterdrücken des Stabes 43 in die Aufnahmehülse 13, so dass die Setscrew 44 einschraubbar wird.

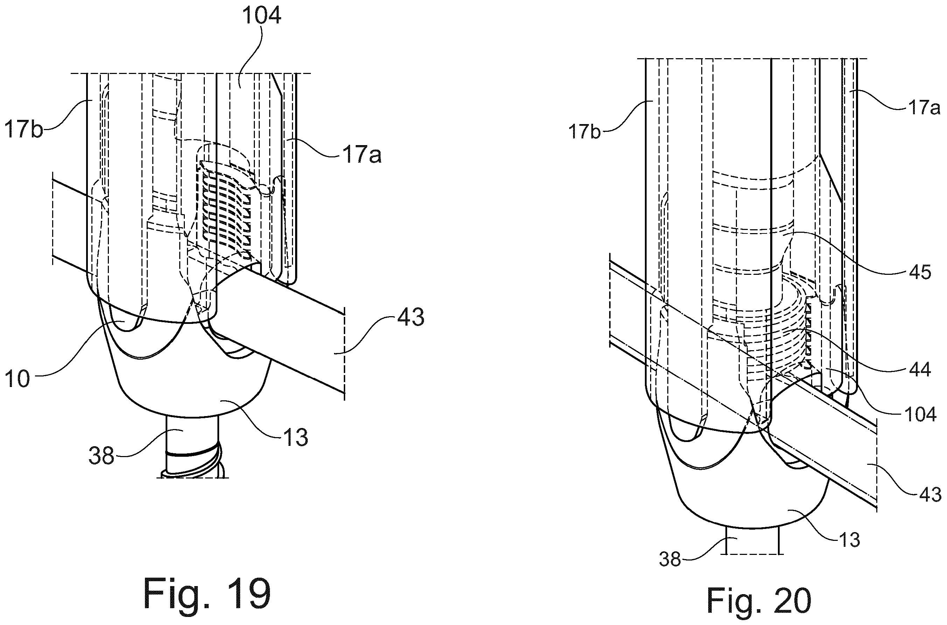

In Figur 19 ist der Zustand des Instruments der provisorischen Fixierung vor Einschrauben einer Setscrew 44 zu sehen. Figur 20 zeigt, ähnlich wie der Einschraubvorgang in Figur 15, das Einschrauben einer Setscrew 44 als Verriegelungselement 44 mittels eines Schraubwerkzeugs 45. Die Setscrew 44 kann zusammen mit dem Schraubwerkzeug 45 durch die Druckeinheit 104 hindurch zur Aufnahmehülse 13 geführt und in deren Innengewinde eingeschraubt werden.

### Bezugszeichenliste

- 1: Koppeleinheit / Koppelelement
- 2: Hülse
- 3: Druckstößel
- 4: Stabdrücker
- 5: Stellmutter
- 6: proximale Basis
- 7: Innengewinde
- 8: Außengewinde
- 9a: Koppelarm
- 9b: Koppelarm
- 10: Koppelstruktur
- 11: Raststruktur
- 12: Gegenraststruktur
- 13: Aufnahmehülse
- 14: Pedikelschraube
- 15: proximale Basis
- 16: Schulter / Anschlag
- 17a: Hülsenarm
- 17b: Hülsenarm
- 18: Fenster / Zwischenraum
- 19: Führungsnut
- 20: Führungsnut
- 21: Führungsvorsprung
- 22: Führungsvorsprung
- 23: Durchlass
- 24: große Halbachse
- 25: kleine Halbachse
- 26: Stab
- 27: Stabaufnahme
- 28: Stützzapfen
- 29: Schraubkopf
- 30: Außengewinde
- 31: Skalierung
- 32: Basis
- 33: Außengewinde
- 34a: Stößelarm
- 34b: Stößelarm
- 35a: Fenster / Zwischenraum
- 35b: Fenster / Zwischenraum
- 36: Stützzapfen
- 37: Schräge
- 38: Gewindeschaft
- 39: Schaftkopf
- 40: Nut / Hinterschnitt
- 41: Gegenkontur / Hinterschnitt
- 42: Klemmstempel
- 43: Längsträger / Stab
- 44: Setscrew
- 45: Schraubwerkzeug
- 46: Vertiefung
- 47: Mitnahmeprofil
- 48: Aufsatzprofil
- 49: Außengewinde
- 50: Ovale Form
- 51: Distaler Abschnitt
- 52: Stabaufnahme
- 53: geriffelte Struktur
- 104: Druckeinheit

## Patentansprüche

1. Medizintechnisches Instrument zur provisorischen Fixierung einer polyaxialen Pedikelschraube (14) mit einem Gewindeschaft (38) mit einem proximal daran ausgebildeten Schaftkopf (39), an dem eine Aufnahmehülse (13) drehbar und/oder schwenkbar angeordnet ist, wobei in der Aufnahmehülse (13) ein Klemmstempel (42) auf den Schaftkopf (39) einwirkend gelagert ist, derart, dass durch relatives Verspannen von Aufnahmehülse (13) und Klemmstempel (42) die Aufnahmehülse (13) am Schaftkopf (39) lagefixierbar ist, wobei das Instrument eine Koppeleinheit (1) zum lösbaren Ineingriffbringen mit der Aufnahmehülse (13) und einen Druckstößel (3) zum Ausüben einer Anpresskraft auf den Klemmstempel (42) in distale Richtung aufweist, wobei die Koppeleinheit (1) und der Druckstößel (3) in einer Axialrichtung relativ zueinander bewegbar sind, **dadurch gekennzeichnet, dass** das Instrument eine Hülse (2) aufweist, in der die Koppeleinheit (1) in die Axialrichtung bewegbar angeordnet ist, derart, dass die Koppeleinheit (1) in einer ersten Axialposition mit der Aufnahmehülse (13) koppelbar oder entkoppelbar ist und in einer zweiten Axialposition an der Aufnahmehülse (13) nicht lösbar festgelegt ist, um zusammen mit dem Druckstößel (3) eine provisorische Fixierung der Pedikelschraube (14) zu realisieren.

2. Medizintechnisches Instrument nach Anspruch 1, **dadurch gekennzeichnet, dass** die Koppeleinheit (1) in einem distalen Endbereich eine radial nach innen vorstehende Raststruktur (10, 11) aufweist, zum koppelnden Eingriff in eine dafür vorgesehene Gegenraststruktur (12) der Aufnahmehülse (13) wobei die Raststruktur (10, 11) vorzugsweise in radialer Richtung zur Hülse (2) relativpositionierbar ist, insbesondere infolge von elastischer Verformung der Koppeleinheit (1) in radialer Richtung.

3. Medizintechnisches Instrument nach einem der vorstehenden Ansprüche, **dadurch gekennzeichnet, dass** die Koppeleinheit (1) mindestens einen sich in die Axialrichtung erstreckenden Koppelarm (9a, 9b) aufweist, dessen distaler Endbereich in radialer Richtung positionierbar ist, insbesondere infolge von elastischer Verformung in radiale Richtung.

4. Medizintechnisches Instrument nach Anspruch 2 oder 3, **dadurch gekennzeichnet, dass** der distale Endbereich der Koppeleinheit (1) in der zweiten Axialposition in radialer Richtung an der Hülse (2) anliegt.

5. Medizintechnisches Instrument nach einem der vorstehenden Ansprüche, **dadurch gekennzeichnet, dass** die Koppeleinheit (1) und die Hülse (2) über ein Gewinde (5, 8) miteinander in Eingriff stehen und durch gegenseitiges Verdrehen axialpositionierbar sind.

6. Medizintechnisches Instrument nach einem der vorstehenden Ansprüche, **dadurch gekennzeichnet, dass** die Hülse (2) an ihrem distalen Ende eine Aufnahme (40) für einen Abschnitt (41) der Aufnahmehülse (13) aufweist, beispielsweise in Form einer Nut, eines Vorsprungs oder eines Hinterschnitts (40), vorzugsweise in umfänglicher Richtung ausgebildet.

7. Medizintechnisches Instrument nach einem der vorstehenden Ansprüche, **dadurch gekennzeichnet, dass** die Koppeleinheit (1) mit einem zentralen Durchlass in Axialrichtung versehen ist, in der der Druckstößel (3) axialpositionierbar angeordnet ist.

8. Medizintechnisches Instrument nach einem der vorstehenden Ansprüche, **dadurch gekennzeichnet, dass** der Druckstößel (3) am proximalen Ende eine zylinderförmige Basis (32) aufweist, die frei um die Axialrichtung des Druckstößels (3) drehbar, axialfest mit dem Druckstößel gekoppelt ist und mit der eine Axialpositionierung von Druckstößel (3) und Koppeleinheit (1) relativ zueinander erfolgt.

9. Medizintechnisches Instrument nach einem der vorstehenden Ansprüche, ferner **gekennzeichnet durch** einen Stabdrücker (4), zum Andrücken eines in der Aufnahmehülse (13) der Pedikelschraube (14) zu fixierenden Stabs oder Längsträgers (43) in distaler Richtung, wobei der Druckstößel (3) einen zentralen Durchlass in Axialrichtung aufweist, in dem der Stabdrücker (4) axialpositionierbar ist, wobei der Druckstößel (3) und der Stabdrücker (4) vorzugsweise als einteilige Druckeinheit (104) ausgebildet sind.

10. Medizintechnisches Instrument nach Anspruch 9, **dadurch gekennzeichnet, dass** der Stabdrücker (4) oder die Druckeinheit (104) einen zentralen Durchlass in Axialrichtung, zur um die Axialrichtung drehbaren Aufnahme einer Setscrew (44) und/oder eines Schraubwerkzeugs (45) zur Schraubbetätigung der Pedikelschraube (14) und/oder zur Schraubbetätigung der Setscrew (44) und/oder dass der Stabdrücker (4) oder die Druckeinheit (104) am proximalen Ende einen drehbaren, axialfest gekoppelten Schraubkopf (29) aufweist, mit dem eine Axialpositionierung relativ zur Koppeleinheit (1) erfolgt.

11. Medizintechnisches Instrument nach einem der vorstehenden Ansprüche, **dadurch gekennzeichnet, dass** die Hülse (2) einen zentralen Durchlass in Axialrichtung aufweist, der quer zur Axialrichtung eine im wesentliche ovale oder elliptische Querschnittsform, zur um die Axialrichtung drehbaren Aufnahme eines Exzenters zum Aufspreizen der Hülse (2) und/oder der Koppeleinheit (1), aufweist.

12. Medizintechnisches Instrument nach einem der Ansprüche 3 bis 11, **dadurch gekennzeichnet, dass** der Übergang von dem Koppelarm (9a, 9b) zu der Raststruktur (10, 11) derart ausgebildet ist, dass die radial äußere Seite der Raststruktur (10, 11) gegenüber der radial äußeren Seite des jeweiligen Koppelarms (9a, 9b) radial nach außen vorsteht.

13. Medizinisches Instrument nach einem der vorstehenden Ansprüche, **dadurch gekennzeichnet, dass** die Raststruktur (10, 11) derart in eine von der Hülse (13) ausgeformte Gegenraststruktur (12) eingreift, dass von der Koppeleinheit (1) eine Zugkraft auf die Hülse übertragbar ist.

14. Medizinisches Instrument nach Anspruch 13, **dadurch gekennzeichnet, dass** die Gegenraststruktur (12) zumindest eine Fläche aufweist, die zumindest teilweise in horizontaler Richtung verläuft und zu der distal die Raststruktur (10, 11) anordenbar ist, um die Zugkraft formschlüssig zu übertragen.

15. Medizinisches Instrument nach einem der Ansprüche 13 oder 14, **dadurch gekennzeichnet, dass** die Gegenraststruktur (12) in ihrem distalen Endbereich eine bauchige Form aufweist und/oder zwei entgegengesetzte, in Umfangsrichtung herausstehende Ausnehmungen in Form einer Hinterschneidung aufweist.

## Claims

1. A medical instrument for provisionally fastening a polyaxial pedicle screw (14) having a threaded shank (38) having a shank head (39) formed proximally thereon, wherein a holding sleeve (13) is rotatably and/or pivotally arranged on the shank head (39), wherein a clamping plunger (42) acting on the shank head (39) is mounted in the holding sleeve (13), in such a way that the holding sleeve (13) can be positionally fixed on the shank head (39) by relative tensioning the holding sleeve (13) and the clamping plunger (42), the instrument having a coupling unit (1) for releasably engaging the holding sleeve (13) and a pressure tappet (3) for applying a pressing force to the clamping plunger (42) in the distal direction, the coupling unit (1) and the pressure tappet (3) being movable relative to each other in an axial direction, **characterized in that** the instrument has a sleeve (2) in which the coupling unit (1) is arranged so as to be movable in the axial direction such that the coupling unit (1) can be coupled to or uncoupled from the holding sleeve (13) in a first axial position and is non-detachably fastened to the holding sleeve (13) in a second axial position in order to implement a provisional fastening of the pedicle screw (14) together with the pressure tappet (3).

2. The medical instrument according to claim 1, **characterized in that** the coupling unit (1) has a radially inwardly projecting detent structure (10, 11) in a distal end region for coupling engagement with a mating detent structure (12) of the holding sleeve (13) provided therefor, wherein the detent structure (10, 11) preferably can be positioned relative to the sleeve (2) in the radial direction, in particular due to elastic deformation of the coupling unit (1) in the radial direction.

3. The medical instrument according to any of the preceding claims, **characterized in that** the coupling unit (1) has at least one coupling arm (9a, 9b) extending in the axial direction, the distal end region of which can be positioned in the radial direction, in particular due to elastic deformation in the radial direction.

4. The medical instrument according to claim 2 or 3, **characterized in that** in the second axial position the distal end region of the coupling unit (1) bears against the sleeve (2) in the radial direction.

5. The medical instrument according to any of the preceding claims, **characterized in that** the coupling unit (1) and the sleeve (2) are in engagement with one another via a thread (5, 8) and can be axially positioned by mutual rotation.

6. The medical instrument according to any of the preceding claims, **characterized in that** the sleeve (2) has its distal end provided with a mount (40) for a section (41) of the holding sleeve (13), for example in the form of a groove, a projection or an undercut (40), preferably formed so as to extend in the circumferential direction.

7. The medical instrument according to any of the preceding claims, **characterized in that** the coupling unit (1) is provided with a central passage in the axial direction, in which the pressure tappet (3) is arranged so as to be axially positionable.

8. The medical instrument according to any of the preceding claims, **characterized in that** the pressure tappet (3) has the proximal end provided with a cylindrical base (32) which is freely rotatable about the axial direction of the pressure tappet (3), is coupled to the pressure tappet in an axially fixed manner and with which an axial positioning of pressure tappet (3) and coupling unit (1) relative to one another is achieved.

9. The medical instrument according to any of the preceding claims, further **characterized by** a rod pusher (4) for pressing a rod or longitudinal beam (43) to be fastened in the holding sleeve (13) of the pedicle screw (14) in the distal direction, the pressure pusher (3) having a central passage in the axial direction in which the rod pusher (4) can be axially positioned, the pressure tappet (3) and the rod pusher (4) preferably being formed as a one-piece pressing unit (104).

10. The medical instrument according to claim 9, **characterized in that** the rod pusher (4) or the pressing unit (104) has a central passage in the axial direction for receiving, in a manner so as to be rotatable about the axial direction, a set screw (44) and/or a screw-driving tool (45) for screwing the pedicle screw (14) and/or for screwing the set screw (44), and/or that the rod pusher (4) or the pressing unit (104) has the proximal end provided with a rotatable screw head (29) which is coupled in an axially fixed manner and with which the axial positioning relative to the coupling unit (1) is effected.

11. The medical instrument according to any of the preceding claims, **characterized in that** the sleeve (2) has a central passage in the axial direction which has a substantially oval or elliptical cross-sectional shape transverse to the axial direction for receiving, in a manner so as to be rotatable about the axial direction, an eccentric for expanding the sleeve (2) and/or the coupling unit (1).

12. A medical instrument according to any of the claims 3 to 11, **characterized in that** the transition from the coupling arm (9a, 9b) to the detent structure (10, 11) is formed such that the radially outer side of the detent structure (10, 11) projects radially outwards with respect to the radially outer side of the respective coupling arm (9a, 9b).

13. The medical instrument according to any of the preceding claims, **characterized in that** the detent structure (10, 11) engages in a mating detent structure (12) formed by the sleeve (13) such that a tensile force can be transmitted from the coupling unit (1) to the sleeve.

14. The medical instrument according to claim 13, **characterized in that** the mating detent structure (12) has at least one surface which extends at least partially in the horizontal direction and relative to which the detent structure (10, 11) can be arranged distally in order to transmit the tensile force in a form-fitting manner.

15. The medical instrument according to any of the claims 13 or 14, **characterized in that** the mating detent structure (12) has a bulbous shape in its distal end region and/or has two opposite indentations in the form of an undercut protruding in the circumferential direction.

## Revendications

1. Instrument médical pour la fixation provisoire d'une vis pédiculaire polyaxiale (14) avec une tige filetée (38) avec une tête de tige (39) formée de manière proximale sur celle-ci, sur laquelle une douille de réception (13) est disposée de manière rotative et/ou de manière pivotante, dans lequel un poinçon de serrage (42) est monté dans la douille de réception (13) de manière à agir sur la tête de tige (39), de sorte que, par serrage relatif de la douille de réception (13) et du poinçon de serrage (42), la douille de réception (13) peut être fixée en position sur la tête de tige (39), dans lequel l'instrument présente une unité de couplage (1) pour la mise en prise amovible avec la douille de réception (13) et un poussoir (3) pour exercer une force de pression sur le poinçon de serrage (42) dans la direction distale, dans lequel l'unité de couplage (1) et le poussoir (3) sont déplaçables l'un par rapport à l'autre dans une direction axiale, **caractérisé en ce que** l'instrument présente une douille (2) dans laquelle l'unité de couplage (1) est disposée de manière déplaçable dans la direction axiale, de sorte que l'unité de couplage (1) peut être couplée ou découplée de la douille de réception (13) dans une première position axiale et est fixée de manière non amovible à la douille de réception (13) dans une seconde position axiale afin de réaliser, conjointement avec le poussoir (3), une fixation provisoire de la vis pédiculaire (14).

2. Instrument médical selon la revendication 1, **caractérisé en ce que** l'unité de couplage (1) présente une structure d'encliquetage (10, 11) faisant saillie radialement vers l'intérieur dans une zone d'extrémité distale pour l'engagement de couplage dans une structure de contre-encliquetage (12) de la douille de réception (13) prévue à cet effet, dans lequel la structure d'encliquetage (10, 11) peut être positionnée par rapport à la douille (2), de préférence dans la direction radiale, en particulier à la suite d'une déformation élastique de l'unité de couplage (1) dans la direction radiale.

3. Instrument médical selon l'une quelconque des revendications précédentes, **caractérisé en ce que** l'unité de couplage (1) présente au moins un bras de couplage (9a, 9b) s'étendant dans la direction axiale, dont la zone d'extrémité distale peut être positionnée dans la direction radiale, en particulier à la suite d'une déformation élastique dans la direction radiale.

4. Instrument médical selon la revendication 2 ou 3, **caractérisé en ce que** la zone d'extrémité distale de l'unité de couplage (1) repose contre la douille (2) dans la direction radiale dans la seconde position axiale.

5. Instrument médical selon l'une quelconque des revendications précédentes, **caractérisé en ce que** l'unité de couplage (1) et la douille (2) sont en prise l'une avec l'autre par l'intermédiaire d'un filetage (5, 8) et peuvent être positionnées axialement par rotation mutuelle.

6. Instrument médical selon l'une quelconque des revendications précédentes, **caractérisé en ce que** la douille (2) présente un logement (40) pour une section (41) de la douille de réception (13) à son extrémité distale, par exemple sous forme d'une rainure, une saillie ou une contre-dépouille (40), de préférence formée dans la direction circonférentielle.

7. Instrument médical selon l'une quelconque des revendications précédentes, **caractérisé en ce que** l'unité de couplage (1) est dotée d'un passage central dans la direction axiale, dans lequel le poussoir (3) est disposé de manière à pouvoir être positionné axialement.

8. Instrument médical selon l'une quelconque des revendications précédentes, **caractérisé en ce que** le poussoir (3) présente à l'extrémité proximale une base en forme de cylindre (32) qui peut tourner librement autour de la direction axiale du poussoir (3), qui est couplée de manière fixe axialement avec le poussoir, et avec laquelle un positionnement axial du poussoir (3) et de l'unité de couplage (1) s'effectue l'un par rapport à l'autre.

9. Instrument médical selon l'une quelconque des revendications précédentes, **caractérisé en outre par** un poussoir à barre (4) pour pousser une barre ou une traverse (43) à fixer dans la douille de réception (13) de la vis pédiculaire (14) dans la direction distale, dans lequel le poussoir (3) présente un passage central dans la direction axiale, dans lequel le poussoir à barre (4) peut être positionné axialement, dans lequel le poussoir (3) et le poussoir à barre (4) sont de préférence conçus en tant qu'unité de pression monobloc (104).

10. Instrument médical selon la revendication 9, **caractérisé en ce que** le poussoir à barre (4) ou l'unité de pression (104) présente un passage central dans la direction axiale pour la réception d'une vis de réglage (44) et/ou d'un outil de vissage (45) pouvant tourner autour de la direction axiale pour l'actionnement par vissage de la vis pédiculaire (14) et/ou pour l'actionnement par vissage de la vis de réglage (44) et/ou **en ce que** le poussoir à barre (4) ou l'unité de pression (104) présente à l'extrémité proximale une tête de vis (29) rotative, couplée de manière fixe axialement, avec laquelle s'effectue un positionnement axial par rapport à l'unité de couplage (1).

11. Instrument médical selon l'une quelconque des revendications précédentes, **caractérisé en ce que** la douille (2) présente un passage central dans la direction axiale, qui présente transversalement à la direction axiale une forme de section transversale sensiblement ovale ou elliptique, pour la réception d'un excentrique pouvant tourner autour de la direction axiale pour écarter la douille (2) et/ou l'unité de couplage (1).

12. Instrument médical selon l'une quelconque des revendications 3 à 11, **caractérisé en ce que** la transition entre le bras de couplage (9a, 9b) et la structure d'encliquetage (10, 11) est conçue de sorte que le côté radialement extérieur de la structure d'encliquetage (10, 11) fait saillie radialement vers l'extérieur par rapport au côté radialement extérieur du bras de couplage respectif (9a, 9b).

13. Instrument médical selon l'une quelconque des revendications précédentes, **caractérisé en ce que** la structure d'encliquetage (10, 11) s'engage dans une structure de contre-encliquetage (12) formée par la douille (13) de sorte qu'une force de traction peut être transmise de l'unité de couplage (1) à la douille.

14. Instrument médical selon la revendication 13, **caractérisé en ce que** la structure de contre-encliquetage (12) présente au moins une surface qui s'étend au moins partiellement dans la direction horizontale et par rapport à laquelle la structure d'encliquetage (10, 11) peut être disposée distalement pour transmettre la force de traction par complémentarité de formes.

15. Instrument médical selon l'une quelconque des revendications 13 ou 14, **caractérisé en ce que** la structure de contre-encliquetage (12) présente une forme bombée dans sa zone d'extrémité distale et/ou présente deux évidements opposés en saillie dans la direction circonférentielle, sous forme d'une contre-dépouille.
